# EUROPÄISCHE PATENTSCHRIFT

(11) **EP 1 338 302 B1**
(45) Veröffentlichungstag und Bekanntmachung des Hinweises auf die Patenterteilung: **26.12.2007**
(21) Anmeldenummer: 03003511.7
(22) Anmeldetag: 15.02.2003
(51) Int. Cl.: A61N 2/00, A61N 2/02

(54) **Vorrichtung zur Stimulation der Mikrowellenerzeugung in Organismen**
Device for stimulating the production of microwaves in an organism
Appareil pour stimuler la production de micro-ondes dans un organisme

(30) Priorität: 22.02.2002 DE 10207588
(43) Veröffentlichungstag der Anmeldung: 27.08.2003
(73) Patentinhaber: Warnke United Research & Development GmbH, 66121 Saarbrücken (DE)
(72) Erfinder: Warnke, Ulrich, Dr. rer. nat., 66133 Saarbrücken (DE)
(74) Vertreter: Bernhardt, Winfrid

(56) Entgegenhaltungen:
- EP-A- 0 885 628
- DE-A- 3 244 582

## Beschreibung

Die Erfindung betrifft eine Vorrichtung zur Beaufschlagung von Organismen, insbesondere des menschlichen Körpers, mit Magnetfeldern, mit einer Sendereinrichtung für die Erzeugung eines den Organismus durchsetzenden Magnetfeldes und einer mit der Sendereinrichtung verbundenen Steuereinrichtung, welche Elektroden zum Abgreifen organismuseigener elektrischer Signale aufweist, die zur Verstärkung und Weiterverarbeitung durch die Steuereinrichtung zu Steuersignalen für die Steuerung der Stärke des Magnetfeldes vorgesehen sind.

Eine Magnetfeldapplikationseinrichtung solcher Art geht aus der DE 32 44 582 A1 hervor. Als Einrichtung zur Erzeugung eines das Magnetfeld erzeugenden Grundstroms sind Frequenz- oder/und Wobbelgeneratoren sowie Rauschgeneratoren vor gesehen. Die Ströme dieser Generatoren werden nach Verstärkung weiteren Schaltungen zugeführt, darunter Modulatoren und Multiplikationsschaltungen. Letzteren kann wahlweise zur Modulation ein durch die Elektroden abgegriffenes verarbeitetes Signal zugeführt werden. Verstärkte modulierte Ströme gelangen in zeitlich nacheinander angeregte, zueinander um 90° versetzte Magnetspulen. Durch die vier Spulen wird ein zirkulierendes Magnetfeld erzeugt, das in seiner Zirkulationsfrequenz physiologischen Körperfrequenzen, z.B. Nervenimpulsfrequenzen, angepasst sein kann.

Beim Informationsaustausch innerhalb des Genoms und des Proteoms wie auch zwischen Genom und Proteom spielen Mikrowellen eine wichtige Rolle. Innerhalb der betreffenden Molekülstrukturen des Organismus' ausgesandte, aus mehreren Sinuswellen-Komponenten zusammengesetzte elektromagnetische Wellen lösen sich innerhalb eines Zellgewebes nicht durch Dispersion auf. Vielmehr kommt es im Rahmen der für Organismen typischen Nichtlinearitäten zur Wellenkopplung und zu kohärenten Wellen unter entsprechend konformen Veränderungen der Molekülstrukturen. Mit letzteren Änderungen gehen Variationen der für die Vitalfunktionen der Zellen maßgebenden Reaktionsfähigkeit der Molekülstrukturen einher.

Der vorliegenden Erfindung liegt die Aufgabe zugrunde, eine einfache Vorrichtung zu schaffen, die es ermöglicht, die Mikrowellenerzeugung in Zellinhalts- und Membranstrukturen von Organismen und damit deren Vitalfunktionen anzuregen.

Diese Aufgabe wird durch eine Vorrichtung der eingangs erwähnten Art gelöst, die dadurch gekennzeichnet ist, dass die Sendereinrichtung eine induktivitätsarme Spule aufweist und dass die Steuereinrichtung zum Abgreifen von Potentialimpulsen vorgesehen ist, welche in ihrer Zeitabhängigkeit Nerv-, Muskel- oder Drüsenaktivitäten betreffenden Aktionspotentialen in Zellinhalts- und Membranstrukturen entsprechen, und die Steuereinrichtung ferner einen Integrator zur Verarbeitung der Potentialimpulse zu Steuerimpulsen umfasst, so dass durch die Sendereinrichtung im Organismus impulsweise an die Aktionspotentiale angepasste Potentiale induzierbar sind, die sich den Aktionspotentialen in den Zellinhalts- und Membranstrukturen unter Stimulation von Mikrowellenerzeugung überlagern.

Bei den genannten organismuseigenen Potentialen handelt es sich vorrangig um Potentiale, die sich an Zellmembranen ausbilden. Sowohl eine Schwächung als auch Verstärkung solcher Potentiale durch das überlagerte, erfindungsgemaß induzierte elektrische Feld bzw. Potential führt zu einer Auslösung von Mikrowellen.

Die Elektronen innerhalb der Membran haben bei Anliegen eines Ruhepotentials von 70 mV eine potentielle Energie von dementsprechend 70 meV. Diese Energie entspricht einer Photonenfrequenz von 1,7 x 10¹³ Hz bzw. einer Photonenwellenlänge von 17,6 µm. Durch Depolarisation, wie sie gemäß der Erfindung infolge Überlagerung der Ruhepotentiale mit induzierten Potentialen auftritt, kommt es zur Freisetzung von Photonen, die den oben angegebenen Werten vergleichbare Frequenzen und Wellenlängen aufweisen. Gleichzeitig kommt es zu einer kohärenten Freisetzung von Photonen im Wellenlängenbereich von 5 bis 20 µm, in dem durch die Depolorisation eine auf großen coulombschen Kräften des Ruhepotentials beruhende Einschränkung der Freiheitsgrade der Schwingungen in den Membranen eingelagerter Dipole aufgehoben werden.

Vorteilhaft macht es die Erfindung möglich, tief im Innern von Organismen Mikrowellen zu erzeugen, deren Transport an die betreffenden Stellen im Organismus von außen infolge Absorption von Mikrowellen in dem im Organismus enthaltenen Wasser ausgeschlossen wäre.

Eine Anpassung der induzierten Potentiale erfolgt sowohl in bezug auf die Impulsform als auch die Zeitabstände der Impulse, d.h. in bezug auf die der gesamten Impulsfolge entsprechende Spannungs-Zeit-Funktion. Ein durch die integrierten Impulse gesteuerter Spulenstrom führt zu einem sich ändernden Magnetfeld, das ein Potential induziert, welches im Zeitverhalten dem abgegriffenen Impuls entspricht, indem bei der Induktion eine die vorangehende Integration aufhebende Differentiation nach der Zeit erfolgt.

In weiterer vorteilhafter Ausgestaltung der Erfindung weist die Steuereinrichtung eine elektronische Speichereinrichtung zur Aufzeichnung der vom Organismus abgegriffenen oder/und bearbeiteten, die Magnetfeldstärke steuernden Signale auf. Eine solche Speichereinrichtung eröffnet die Möglichkeit, die Steuersignale für die Steuerung der Magnetfeldstärke zwischenzuspeichern und zeitversetzt zu ihrer Aufzeichnung auszugeben und damit die Beaufschlagung des Organismus' mit dem Magnetfeld zeitversetzt zum Abgriff der Potentiale am Organismus durchzuführen.

Andererseits kann die Steuereinrichtung zur Ausgabe der Steuersignale gleichzeitig mit dem Abgreifen von Potentialen am Organismus vorgesehen sein, wobei die Elektroden organismuseigene Potentiale in Überlagerung mit durch die Sendereinrichtung erzeugten endogenen Potentialen als Interferenzrückkopplungssignale abgreifen.

In zweckmäßiger Ausgestaltung der Erfindung kann die Steuerung zwischen gleichzeitiger und zeitversetzter Ausgabe der Steuersignale umschaltbar sein und daher mit oder ohne direkte Rückkopplung arbeiten.

In der bevorzugten Ausführungsform der Erfindung weist die Sendeeinrichtung eine Spule auf, die für den sich schnell ändernden gesteuerten Spulenstrom einen nur geringen induktiven Widerstand darstellt. Zweckmäßig ist die Windungszahl kleiner als fünfundzwanzig bei einer axialen Länge von 0,5 bis 1,5 cm und einem Durchmesser von 10 bis 20 cm.

Die Erfindung soll nun anhand eines Ausführung beispiels und der beiliegenden, sich auf dieses Ausführungsbeispiel beziehenden Zeichnungen näher erläutert werden. Es zeigen:
- Fig. 1: eine Schemadarstellung einer erfindungsgemäßen Vorrichtung zur Stimulation der Mikrowellenerzeugung in Organismen, und

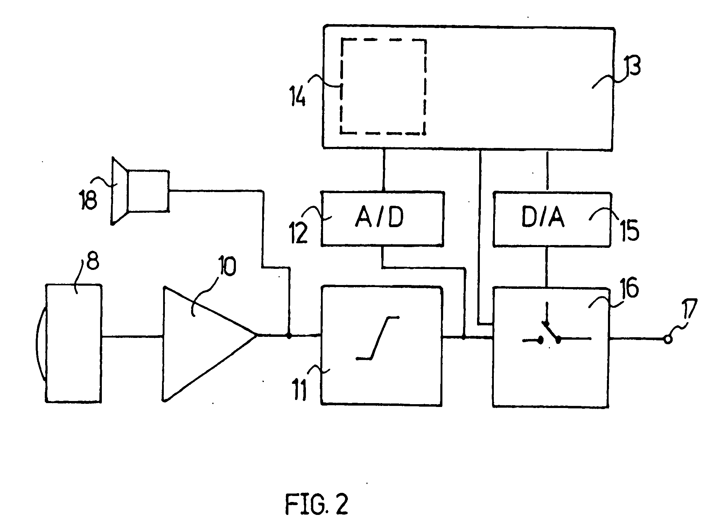

- Fig. 2: eine Schemadarstellung einer in der Vorrichtung von Fig. 1 verwendeten Steuereinrichtung.

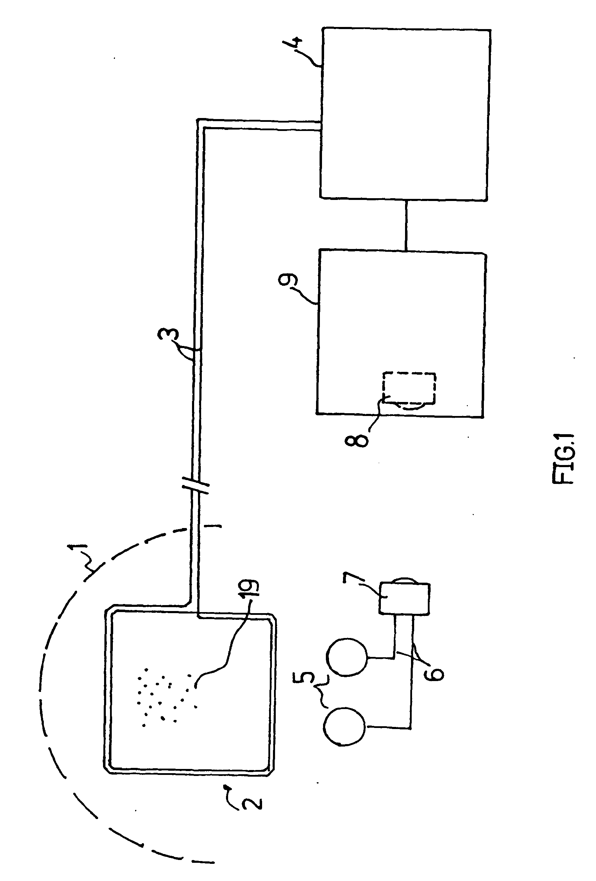

Mit der Bezugszahl 1 ist in Fig. 1 ein menschlicher Oberkörper angedeutet, an dessen Rücken eine Spule 2 angeordnet ist.

Die schematisch dargestellte Spule 2 weist eine nur geringe Induktivität auf. Bei einer axialen Länge von 1,2 cm und einem Durchmesser von etwa 10 bis 20 cm hat die Spule 2 weniger als fünfundzwanzig Windungen.

Die Spule 2 ist über Leitungen 3 mit einer Stromversorgungseinheit 4 verbunden, welche eine nicht gezeigte Leistungssteuereinrichtung enthält, über die sich der Spulenstrom entsprechend einer gewünschten Strom-Zeit-Funktion steuern lässt.

Das Bezugszeichen 5 weist auf zwei Elektroden hin, von denen die Person, an deren Rücken die Spule 2 angeordnet ist, eine mit der linken und die andere mit der rechten Hand ergreifen kann.

Die Elektroden 5 sind über Leitungen 6 mit einer Infrarotsendeeinrichtung 7 verbunden. Die Infrarotsendeeinrichtung 7 überträgt drahtlos ein Signal, das einer über den Elektroden 5 liegenden, am Körper abgegriffenen Spannung entspricht. Das Signal wird von einer Infrarotempfängereinrichtung 8 empfangen, welche in eine mit der Stromversorgungseinheit 4 verbundene Steuereinheit 9 eingebaut ist.

Die Infrarotempfängereinrichtung 8 liefert ausgangsseitig ein der Spannung über den Elektroden 5 entsprechendes Spannungssignal, das einem Verstärker 10 zugeführt wird. Der Ausgang des Verstärkers 10 steht in Verbindung mit einem Integrator 11. An den Ausgang des Integrators ist ein Analogdigitalwandler 12 angeschlossen, der mit einer die digitalen Ausgangssignale des Wandlers empfangenden Rechnereinheit 13 in Verbindung steht.

Die Rechnereinheit 13 enthält in üblicher Weise einen Prozessor, Speicher- sowie Ein- und Ausgabebausteine. Eine Strichlinie deutet einen durch die Programmierung der Rechnereinheit 13 gebildeten Pufferspeicher an.

An einen Datenausgang der Rechnereinheit 13 ist ein Digitalanalogwandler 15 angeschlossen, dessen Analogausgang mit einer durch die Rechnereinheit 13 steuerbaren Schaltereinrichtung 16 verbunden ist, welche wahlweise den Analogausgang des Digitalwandlers 16 oder den Ausgang des Integrators 11 mit einem Steuerausgang 17, welcher der genannten Leistungssteuereinrichtung der Stromversorgungseinheit 4 eine Führungsgröße zur Verfügung stellt.

Der Ausgang des Verstärkers 10 ist mit einer akkustischen Signalgebereinrichtung 18 verbunden.

Im folgenden wird die Funktionsweise der vorangehend beschriebenen Vorrichtung erläutert.

Ergreift eine Person die Elektroden 5 mit ihren Händen, so liegen zwischen den Elektroden körpereigene Potentialimpulse mit wechselnder Polarität an, die auf Nerv- und Muskelaktivitäten zurückzuführen sind.

Die Infrarotsendeeinrichtung 7 überträgt diese Impulse zu der Infrarotempfängereinrichtung 8, an deren Ausgang die durch die Elektroden 5 abgegriffenen Potentialimpulse als Analogsignale anliegen.

In der nachfolgenden, den Verstärker 10 und den Integrator 11 umfassenden Impulsbearbeitungseinrichtung erfolgen eine Signalverstärkung und eine Integration.

Die bearbeiteten Impulse können in einem ersten Betriebsmodus über die Schaltereinrichtung 16 direkt an den Steuerausgang 17 gelegt werden, um den Strom der Stromversorgungseinheit 4 entsprechend der Form der verarbeiteten Impulse bzw. entsprechend der durch die integrierte Impulsfolge gebildeten Spannungs-Zeit-Funktion zu steuern.

Der in solcher Weise gesteuerte Spulenstrom erzeugt über die induktivitätsarme Spule 2 ein sich entsprechend änderndes Magnet- bzw. Induktionsfeld, wobei im Körper Potentialimpulse proportional der Ableitung dB/dt, d.h. der Induktion B nach der Zeit t induziert werden. Die im Integrator 11 durchgeführte Integration des am Körper abgegriffenen Spannungssignals wird damit gewissermaßen rückgängig gemacht. Infolge der Proportion zwischen dem Spannungssignal, dem Spulenstrom und der Induktion B weisen die induzierten Impulse die gleiche Form wie die körpereigenen Impulse auf.

In dem ersten Betriebsmodus, in welchem der Ausgang des Integrators 11 über die Schaltereinrichtung 16 direkt mit dem Steuerausgang 17 der Steuereinheit 9 verbunden ist, empfangen die Elektroden 5 neben den körpereigenen Potentialimpulsen auch die diesen überlagerten endogenen Impulse, welche auf die Induktion im Körper zurückzuführen sind. Das heißt, in diesem Betriebsmodus liegt an den Elektroden 5 ein Interferenzrückkopplungssignal an.

In einem zweiten Betriebsmodus ist der Steuerausgang 17 der Steuereinheit 9 durch die Schaltereinrichtung 16 sowohl vom Ausgang des Integrators 11 als auch vom Ausgang des Digitalanalogwandlers 15 getrennt. Das am Ausgang des Integrators 11 anliegende Signal wird durch den Analogdigitalwandler 12 digitalisiert und der Rechnereinheit 13 zugeführt, welche das über einen vorbestimmten Zeitraum anfallende digitale Signal in dem Pufferspeicher 14 aufzeichnet.

In einem dritten Betriebsmodus, in welchem der Steuerausgang 17 über die Schaltereinrichtung mit dem Ausgang des Digitalanalogwandlers verbunden ist, kann das aufgezeichnete Signal nun zeitversetzt zur Aufzeichnung durch die Rechnereinheit 13 abgerufen und über den Digitalanalogwandler 15 dem Steuerausgang 17 zugeführt werden.

Wie vorangehend beschrieben, werden im Körper nun wieder Impulse induziert, welche die gleiche Form wie durch den Körper selbst erzeugte und durch die Elektroden 5 abgreifbare Impulse aufweisen.

Beispielsweise können an den Elektroden 5 unter Interferenzrückkopplung anliegende Signale über 0,5 s und anschließend ohne Rückkopplung anliegende Signale über 1,5 s gespeichert werden, wobei sich die Kaskade gespeicherter Signale dann mehrmals abrufen und dem Steuerausgang 17 zuführen lässt. Über die akkustische Signalgebereinrichtung wird das Geschehen hörbar.

Indem die induzierten Impulse die Form der körpereigenen Impulse aufweisen und auch in ihren Zeitabständen den körpereigenen Impulsen entsprechen, kommt es zwischen den induzierten Impulsen und körpereigenen Impulsen zu Überlagerungen, wobei sich Feldstärken addieren oder subtrahieren. Bei solchen Polarisationen bzw.

Depolarisationen werden Mikrowellen freigesetzt, welche dem Informationstransfer innerhalb des Genoms und innerhalb des Proteoms sowie auch zwischen Genom und Proteom dienen.
Alle Betriebsmoden können gemischt werden, so dass neben vorabgespeicherten Signalen auch Interferenzrückkopplungssignale in Echtzeit kurzfristig eingeschoben werden können.

## Patentansprüche

1. Vorrichtung zur Beaufschlagung von Organismen, insbesondere des menschlichen Körpers (1), mit Magnetfeldern, mit einer Sendereinrichtung (2-4) für die Erzeugung eines den Organismus durchsetzenden Magnetfeldes (19) und einer mit der Sendereinrichtung (2-4) verbundene Steuereinrichtung (9) zur Steuerung der Stärke des Magnetfeldes (19), welche Bektraden (5) zum Abgreifen organismuseigener elektrischer Signale aufweist, die zur Verstärkung und Weiterverarbeitung durch die Steuereinrichtung zu Steuersignalen für die Steuerung der Stärke des Magnetfeldes vorgesehen sind,
**dadurch gekennzeichnet,**
**dass** die Sendereinrichtung (2-4) eine induktivitätsame spule (2) aufweist, und dass die Steuereinrichtung (9) zum Abgreifen von Potentialimpulsen vorgesehen ist, welche in ihrer Zeitabhängigkeit Nerv-, Muskel- oder Drüsenaktivitäten betreffenden Aktionspotentialen in Zellinhalts- und Membrantrukturen entsprechen, und die Steuereinrichtung (9) ferner einen Integrator (11) zur Verarbeitung der Potentialimpulse zu Steuerimpulsen umfasst, so dass durch die Sendereinrichtung (2-4) im organismus impulsweise an die Aktionspotentiale angepasste Potentiale induzierbar sind, die sich den Aktionspotentialen in den Zellinhalts- und Membranstrukturen unter Stimulation von Mikrowellenerzeugung Überlagern.

2. vorrichtung nach Anspruch 1,
**dadurch gekennzeichnet,**
**dass** die Steuereinrichtung (9) eine elektronische Speichereinrichtung (13, 14) zur Aufzeichnung der am Organismus abgegriffenen und bearbeiteten Potentialimpulse aufweist.

3. Vorrichtung nach Anspruch 2
**dadurch gekennzeichnet,**
**dass** die Steuereinrichtung (9) zur Ausgabe der Steuersignale zeitversetzt zu Ihrer Aufzeichnung vorgesehen ist.

4. Vorrichtung nach einem der Ansprüche 1 bis 3,
**dadurch gekennzeichnet,**
**dass** die Steuereinrichtung (9) zur Ausgabe der Steuersignale gleichzeitig mit dem Abgreifen von Potentialen am Organismus vorgesehen ist, wobei die Elektroden (5) organismuseigene Potentiale in Überlagerung mit durch die Sendereinrichtung (2-4) erzeugten endogenen Potentialimpulsen als Interferenzrückkopplungssignal abgreifen.

5. Vorrichtung nach Anspruch 4.
**dadurch gekennzeichnet,**
**dass** die Steuereinrichtung (9) zwischen zur Aufzeichnung gleichzeitiger und zeitversetzter Ausgabe der Steuersignale umschaltbar ist.

6. Vorrichtung nach einem der Ansprüche 1 bis 5,
**dadurch gekennzeichnet,**
**dass** die Spule (2) maximal fünfundzwanzig Windungen bei einer axialen Länge von 0,5 bis 1,5 cm und einem Durchmesser von 10 bis 20 cm aufweist.

## Claims

1. Device for applying magnetic fields to organisms, in particular the human body (1), having a transmitter (2-4) for generating a magnetic field (19) permeating the organism and a controller (9) connected to the transmitter (2-4) for controlling the strength of the magnetic field (19), which comprises electrodes (5) for tapping intra-organism electrical signals that are intended for amplification and further processing by the controller in order to form control signals for controlling the strength of the magnetic field, **characterized in that** the transmitter (2-4) comprises a low-inductance coil (2), and **in that** the controller (9) is intended to tap potential pulses which correspond in terms of their time dependence to action potentials affecting nerve, muscle or gland activities in cell content and membrane structures, and the controller (9) furthermore comprises an integrator (11) for processing the potential pulses in order to form control pulses, so that the transmitter (2-4) can induce potentials pulse-wise adapted to the action potentials in the organism, which are superimposed on the action potentials in the cell content and membrane structures so as to stimulate microwave generation.

2. Device according to Claim 1, **characterized in that** the controller (9) comprises an electronic memory (13, 14) for recording the potential pulses tapped from the organism and processed.

3. Device according to Claim 2, **characterized in that** the controller (9) is intended to output the control signals with a time lag from their recording.

4. Device according to one of Claims 1 to 3, **characterized in that** the controller (9) is intended to output the control signals simultaneously with the tapping of potentials from the organism, the electrodes (5) tapping intra-organism electrical signals in superposition with endogenous potential pulses generated by the transmitter (2-4) as an interference feedback signal.

5. Device according to Claim 4, **characterized in that** the controller (9) can be switched over between simultaneous and time-lag output of the control signals with respect to the recording.

6. Device according to one of Claims 1 to 5, **characterized in that** the coil (2) comprises at most twenty-five turns with an axial length of from 0.5 to 1.5 cm and a diameter of from 10 to 20 cm.

## Revendications

1. Dispositif pour soumettre des organismes, en particulier le corps humain (1) à des champs magnétiques, avec un dispositif émetteur (2-4) pour la génération d'un champ magnétique (19) traversant l'organisme et un dispositif de commande (9) relié au dispositif émetteur (2-4) pour commander l'intensité du champ magnétique (19), qui présente des électrodes (5) pour capter des signaux électriques propres au corps, qui sont destinés à être renforcés et traités ultérieurement par le dispositif de commande en signaux pour la commande de l'intensité du champ magnétique, **caractérisé en ce que** le dispositif émetteur (2-4) présente une bobine de faible induction (2) et **en ce que** le dispositif de commande (9) est prévu pour capter les impulsions de potentiel, dont la variation dans le temps correspond à des potentiels d'action dans des structures des constituants cellulaires et membranaires, relatifs à des activités des nerfs, des muscles ou des glandes, et le dispositif (9) comprend en outre un intégrateur (11) pour la transformation des impulsions de potentiel en impulsions de commande, de telle manière que le dispositif émetteur (2-4) peut induire dans l'organisme, par des impulsions, des potentiels adaptés aux potentiels d'action, qui se superposent aux potentiels d'action dans les structures des constituants cellulaires et membranaires avec une stimulation par une génération de micro-ondes.

2. Dispositif selon la revendication 1, **caractérisé en ce que** le dispositif de commande (9) présente un dispositif d'accumulation électronique (13, 14) destiné à enregistrer les impulsions de potentiel captées sur l'organisme et traitées.

3. Dispositif selon la revendication 2, **caractérisé en ce que** le dispositif de commande (9) est prévu pour sortir les signaux de commande de manière décalée dans le temps par rapport à leur enregistrement.

4. Dispositif selon l'une quelconque des revendications 1 à 3, **caractérisé en ce que** le dispositif de commande (9) est prévu pour sortir les signaux de commande en même temps que le captage des potentiels sur l'organisme, les électrodes (5) captant les potentiels propres aux corps en superposition avec les impulsions de potentiel endogènes produites par le dispositif émetteur (2-4) comme signal d'interférence de rétroaction.

5. Dispositif selon la revendication 4, **caractérisé en ce que** le dispositif de commande (9) peut être commuté entre la sortie des signaux de commande de manière simultanée et décalée dans le temps par rapport à l'enregistrement.

6. Dispositif selon l'une quelconque des revendications 1 à 5, **caractérisé en ce que** la bobine (2) présente au maximum vingt-cinq spires à une longueur axiale de 0,5 à 1,5 cm et un diamètre de 10 à 20 cm.
